# EUROPÄISCHE PATENTANMELDUNG

(11) **EP 1 726 260 A2**
(43) Veröffentlichungstag der Anmeldung: **29.11.2006**
(21) Anmeldenummer: 06114259.2
(22) Anmeldetag: 19.05.2006
(51) Int. Cl.: A61B 5/16, A61B 3/113, A61B 5/18

(54) **Verfahren und Vorrichtung zum Bestimmen Neurologischer Beeinträchtigungen**

(30) Priorität: 26.05.2005 DE 102005024796
(71) Anmelder: Oculometrics AG, 8006 Zürich (CH)
(72) Erfinder: Muser, Markus, H., 80006, Zürich (CH); Schmitt, Kai-Uwe, 8006, Zürich (CH)
(74) Vertreter: Stocker, Kurt

(57) **Zusammenfassung**

Hier geht es um ein Verfahren zum Bestimmen neurologischer Beeinträchtigungen. Dabei wird eine Augenbewegung einer zu testenden Person mittels eines optischen Stimulus (S, S', S") stimuliert, die Augenbewegung ermittelt und in ein entsprechendes elektronisches Signal umgesetzt. Aus der Augenbewegung wird nach einem Vergleich ein Testsignal (R) gewonnen. Nun wird ein in seiner Position veränderlicher Stimulus (S, S', S") verwendet, dem das Auge (4) folgen soll. Das Testsignal (R) wird dann aus dem Vergleich der Bewegung des Stimulus (Sₐ) mit der Bewegung (M) des Auges (4) gewonnen. Dementsprechend umfasst eine zugehörige Vorrichtung eine ein stimulierendes Signal (S, S', S") abgebende Einrichtung (1, 6, 7), eine die Augenbewegung beobachtende und ein entsprechendes Ausgangssignal liefernde Einrichtung (3) sowie eine Vergleichs- und Auswerteeinrichtung (6). Der ein stimulierendes Signal abgebenden Einrichtung (1, 6, 7) ist ein Bewegungssignal zuführbar und der Vergleichs- und Auswerteeinrichtung (6) ist sowohl das genannte Ausgangssignal als auch das Bewegungssignal zuführbar. Die daraus gewonnenen Unterschiede bilden das Testsignal (R).

## Beschreibung

Die Erfindung bezieht sich auf ein Verfahren zum Bestimmen neurologischer Beeinträchtigungen, bei der eine Augenbewegung einer zu testenden Person stimuliert wird, die Augenbewegung ermittelt und in ein entsprechendes elektronisches Signal umgesetzt wird und aus der Augenbewegung nach einem Vergleich ein Testsignal gewonnen wird.

Ein Verfahren und eine Vorrichtung der eingangs genannten Art ist aus dem US-Patent Nr. 4,889,422 bekannt geworden. Es geht dabei im wesentlichen um die Feststellung einer Dyslexie, d.h. einer Beeinträchtigung beim Lesen von Texten. Dem Patienten wird demzufolge ein feststehender schriftlicher Text zu lesen gegeben, den er mit seinen Augen abtastet. Aus der Art, wie der Patient den Text liest, d.h. ob er etwa Verständnisschwierigkeiten hat und deshalb an den Ausgangspunkt einer Zeile zurückkehrt od.dgl., wird auf Grund von Erfahrungswerten auf seine Krankheit geschlossen.

Dyslexie ist ein relativ seltenes Syndrom, wo aber in bekannter Weise eine gewisse Notwendigkeit besteht, Einblick in neurologische Beeinträchtigungen zu nehmen, ist die Frage der Fahrtüchtigkeit eines Fahrzeuglenkers. Dazu wurde in verschiedenen Dokumenten bereits Vorschläge gemacht, beispielsweise in der WO 89/07537. Allen diesen Systemen ist gemeinsam, dass sie relativ langsam und ungenau arbeiten; insbesondere ist es damit schwierig, schnell ein brauchbares Testsignal zu gewinnen, das Auskunft über den Zustand des Fahrers gibt. Daher eignen sich diese Systeme weder für die laufende Kontrolle von Autofahrern durch die Strassenpolizei, noch für die Verwendung im Fahrzeug selbst, weil das Testergebnis so spät erhalten wird, dass eine wirksame Vermeidung von Unfällen kaum erzielt werden kann.

Der Erfindung liegt daher die Aufgabe zugrunde, ein Verfahren der eingangs genannten Art so auszubilden, dass eine rasche Aussage über den Zustand der Person bzw. über ihre neurologische Beeinträchtigung erhalten werden kann, wobei hier der Ausdruck "neurologisch" in einem sehr allgemeinen Sinne verstanden werden soll, der nämlich sowohl physische Beeinträchtigungen (etwa Ermüdung z.B. der Muskeln) als auch physisch/neurologische Beeinträchtigungen umfassen soll.

Dabei sei nur erwähnt, dass die heute denkbaren neurologischen Beeinträchtigungen von sehr umfangreicher Art sein können. Es geht hier nicht nur um die durch Alkoholgenuss entstandenen Beeinträchtigungen, sonder auch um diejenigen, welche durch Medikamenten- oder Drogeneinnahme, durch Krankheit oder einfach durch Müdigkeit und Erschöpfung verursacht sind. Alle diese Beeinträchtigungen können zu Unfällen führen, wobei einzelne, wie etwa die Müdigkeit, gar nicht durch chemische Tests, wie die bekannten Alkoholtests, bestimmt werden können. Ein weiteres Gebiet betrifft etwa die Messung dessen, was heute gerne als "Fitness" bezeichnet wird, also etwa der Erschöpfung bei zu ausgedehntem Training von Sportlern (im allgemeinsten Sinn), aber auch bei Übungen von Rekruten beim Militär, wobei es ja immer wieder vorgekommen ist, dass die Leistungsfähigkeit der Rekruten von den Vorgesetzten falsch eingeschätzt wurde und dies zu Schäden, ja sogar zum Tode der überforderten Person führte.

Erfindungsgemäss wird die obige Aufgabe dadurch gelöst, dass ein in seiner Position veränderlicher Stimulus verwendet wird, dem das Auge folgen soll, und dass das Testsignal aus dem Vergleich der Bewegung des Stimulus mit der Bewegung des Auges gewonnen wird.

Statt also einen ortsfesten (Lese-)Stimulus zu verwenden, wie beim Stand der Technik, wird ein beweglicher Stimulus eingesetzt und das Auge gezwungen, dieser Bewegung zu folgen. Je nach physisch/psychischem Zustand (Ermüdung und/oder Erschöpfung etwa ist sowohl ein physischer als auch ein psychisch/neurologischer Vorgang) der jeweiligen Testperson wird das Nachfolgen der Augenbewegung unterschiedlich ausfallen.

Es könnte sein, dass die Bewegungsmuster überhaupt voneinander abweichen, weil sich die Testperson einfach nicht mehr auf die Bewegung des Stimulus zu konzentrieren vermag. Im allgemeinen aber wird es so sein, dass das Auge nicht mehr mit der erforderlichen Geschwindigkeit bzw. Beschleunigung folgen kann, und in diesem Falle ist es günstig, wenn bei dem Vergleich der Zeitabstand zwischen den beiden Bewegungen ermittelt und für das Testsignal herangezogen wird.

Um eine Abweichung der Augenbewegung quer zur Bewegungsrichtung des die Position wechselnden optischen Stimulus und damit Verfälschungen bei der Auswertung zu vermeiden, ist es vorteilhaft, wenn der optische Stimulus relativ kleinflächig, insbesondere im wesentlichen punktförmig ist.

Die Erfindung bezieht sich schliesslich auch auf eine Vorrichtung zur Durchführung des oben dargelegten Verfahrens.

Weitere Einzelheiten der Erfindung ergeben sich aus der nachfolgenden Beschreibung eines in der Zeichnung schematisch dargestellten Ausführungsbeispieles. Es zeigen:

Fig. 1 ein erstes Ausführungsbeispiel einer erfindungsgemässen Vorrichtung; die

Fig. 2 und 3 Diagramme zur Erläuterung des Unterschiedes des Vorgehens nach der vorliegenden Erfindung und der des Standes der Technik, wobei ein Detail X der Fig. 2 auch in grösserem Massstab dargestellt ist; und

Fig. 4 zeigt ein weiteres erfindungsgemässes Ausführungsbeispiel.

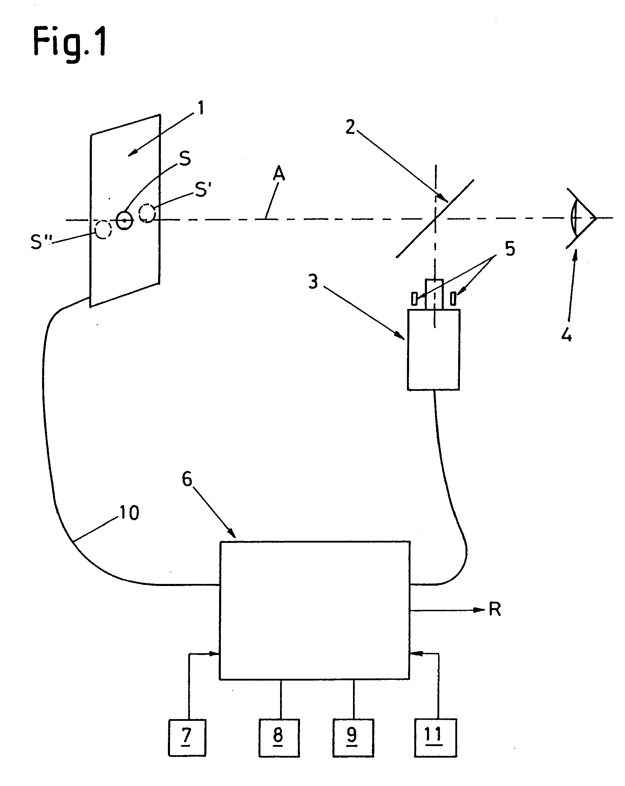

In Fig. 1 ist das Auge 4 einer auf Fahrtüchtigkeit zu testenden Person dargestellt. Diese Person wird dazu aufgefordert (oder in entsprechender Weise genötigt), entlang einer Blickachse A auf einen zu dieser Achse A senkrecht liegenden Bildschirm 1, ein Display od.dgl. zu blicken. Ein solcher Bildschirm 1 kann in einer Testkabine angeordnet sein, es könnte aber auch ein durchsichtiges LCD-Display an der Windschutzscheibe eines Fahrzeuges zur in Zeitabständen erfolgenden Testung des Fahrers so vorgesehen sein, dass bei einem Test die Aufmerksamkeit des Fahrers nicht zu sehr vom Verkehr abgelenkt wird. In Fig. 1 ist dieser Bildschirm 1 zur Verdeutlichung der darauf ersichtlichen Darstellung etwas aus der zur Achse A senkrechten Ebene herausgedreht.

Auf dem Bildschirm 1 od.dgl. ist ein, vorzugsweise kleinflächiger, z.B. von einem Kreis, einem Viereck oder auch einem Buchstaben oder ein Wort, insbesondere einem Punkt, gebildeter, optischer Stimulus S abgebildet, der aus der mit vollen Linien dargestellten Position S in eine Position S' oder S" zu springen vermag. Es ist klar, dass dies beispielsweise dadurch geschehen kann, dass an den Positionen S, S', S" je eine Lichtquelle, wie eine Leuchtdiode, vorgesehen ist, wobei diese Lichtquellen abwechselnd aufleuchten. Eine solche Lösung ist als vereinfachte Lösung bevorzugt, wobei die Lichtquellen beispielsweise als Leuchtdioden an einem "Bildschirm" ausgebildet sein können. In diesem Falle entsprächen die Positionen S, S', S" je einer Leuchtdiode.

Grundsätzlich ist man jedenfalls bei der Auswahl der Art und Form des Stimulus und seiner Positionen relativ frei. Beispielsweise wäre es denkbar, dass sich der Punkt S nicht (nur) horizontal sondern vertikal oder horizontal und vertikal usw. bewegt (X- und Y-Richtung). Dies ist gerade bei Verwendung eines Bildschirmes, d.h. einer zweidimensionalen Darstellungsfläche, leicht und sehr variabel möglich. Es ist aber vorteilhaft, wenn der Stimulus nach jeder Auslenkung, etwa in die Position S' oder S" in eine "Null-Position" zurückkehrt, welche die Position S oder auch eine andere sein kann.

Wenn also durch diesen Positionswechsel des Kreises oder Punktes S eine Bewegung simuliert wird, so folgt das Auge 4 der Testperson dieser Bewegung mit einer mehr oder minder grossen Verzögerung. Selbstverständlich würde eine solche Folgebewegung auch dann erfolgen, wenn sich ein relativ grossflächiges Bild am Bildschirm 1 bewegte. Die Gefahr dabei ist nur, dass das Auge dann sich auch abwechselnd auf verschiedene Punkte des Bildes, also quer zur Bewegungsrichtung des Stimulus S folgt und dadurch das Testergebnis verfälscht werden könnte.

Die Bewegung des Stimulus S könnte etwa durch eine mit dem Bildschirm 1 oder einer auf diesen Bildschirm projizierenden Projektionsvorrichtung verbundenen astabile Vorrichtung gesteuert werden, etwa (z.B. bei nur zwei Positionen S und S', S' und S" oder S und S") einem astabilen Multivibrator. Da aber die Bewegung des Auges 4 beobachtet, in entsprechende Signale umgesetzt und dann ausgewertet werden soll, ist es vorteilhaft, wenn dies nicht von einem separaten Gerät aus erfolgt, dessen Ausgangssignale dann auch einer Auswerteeinrichtung 6 zugeführt werden müssten, sondern von einem einzigen Kontrollgerät 6 aus, das zweckmässig von einem Computer gebildet ist. Nun wurde bereits gesagt, dass es an sich denkbar wäre, für den Stimulus S nur zwei unterschiedliche Positionen vorzusehen. In einem solchen Fall - und noch dann bei regelmässigem Hin- und Herspringen des Stimulus S - wäre die Gefahr gross, dass die Testperson den Takt der Bewegung des Stimulus erfasst und die Augen automatisch im selben Takt bewegt. Deshalb ist es nicht nur vorteilhaft, mehr als zwei Positionen (es können auch mehr als drei sein und die Bewegung kann auch fliessend erfolgen), sondern wenn zusätzlich der Ort (S, S', S") und/oder die Dauer der Darstellung des Kreises, Punktes od.dgl. an einem Ort durch einen Zufallsgenerator 7 zu bestimmen, der mit dem Computer oder Prozessor 6 verbunden ist. In einem konkreten Beispiel bewegte sich der Stimulus zwischen neun verschiedenen Positionen hin und her.

Dabei sollte der Abstand der Positionen zweckmässig nicht zu klein gewählt werden, wenn man davon ausgeht, dass etwa ein Abstand, welcher einer Sehwinkelveränderung des Auges von 0,2° bis 03° entspricht, kaum mehr wahrgenommen wird. Deshalb ist es bevorzugt, wenn die Abstände der Positionen des Stimulus S, S', S" einer Sehwinkelveränderung des Auges 4 von wenigstens 3°, insbesondere von wenigstens 5°, z.B. 10°, entsprechen.

Eine zusätzliche Möglichkeit ergibt sich dadurch, dass man gegebenenfalls den Stimulus mit unterschiedlichen Kontrasten verbindet, beispielsweise indem ein stimulierenden Leuchtpunkt mit unterschiedlicher Helligkeit strahlt. In diesem Falle wäre es durchaus denkbar, die Reaktion der Pupille (Pupillenvergrösserung - Pupillenverkleinerung) zu messen, was gerade bei unter Drogen stehenden Personen einen deutlichen Hinweis liefern kann. Die Messung der Pupillendimension ist im Stande der Technik an sich bekannt, doch lässt sich gerade bei Verwendung einer Kamera mit einer Bildauswerte-Software diese Dimension leicht bestimmen. Diese Möglichkeit ist auch unabhängig von der Bewegung eines Leuchtpunktes od.dgl. anwendbar.

Eine andere Art der Kontrastveränderung kann darin bestehen, dass der stimulierende Punkt auf einem sich verändernden Hintergrund dargestellt wird. Beispielsweise wird als Hintergrund ein Punktmuster (oder ein anderes Bild) gewählt, welches sich noch vor dem Erscheinen oder vor der Bewegung des stimulierenden Bildes oder Punktes oder im Augenblick der Lageveränderung des stimulierenden Bildes sich zu bewegen beginnt. Zusätzlich oder alternativ kann mit jedem Stellungswechsel des Stimulus ein veränderter, also andersartiges oder anders bewegtes, Hintergrundmuster gezeigt werden, um so automatische Bewegungsabläufe des Auges, die das Messergebnis verfälschen könnten, möglichst zu stören. Selbstverständlich kann aber auch ein Hintergrundmuster (bewegt, unbewegt oder bei jeder Stellung des Stimulus sich verändernd) von Vorteil sein, weil es den Probanden zu einer visuellen Differenzierung zwingt.

Während also das Auge 4 der Bewegung des Stimulus folgt, wird die Augenbewegung in an sich bekannter Weise abgetastet bzw. aufgenommen. Die Aufnahme der Augenbewegung ist in der Technik - etwa des Fokussierens optischer Systeme, wie einer Photokamera - an sich bekannt (vgl- z.B. DE-A-42 15 523 oder JP-A-6-331880), und es können diese Systeme auch im Rahmen der vorliegenden Erfindung angewandt werden. Bevorzugt ist es allerdings, wenn eine Videokamera 3 verwendet wird. Dabei ist es besonders bevorzugt, wenn die Videokamera 6 als Hochgeschwindigkeitskamera mit einer grösseren Bildfrequenz als 100 Bildern/s, vorzugsweise mindestens 250 Bilder/s und insbesondere in einem Bereich von 400 bis 600 Bildern/s, z.B. 500 Bildern/s ausgebildet ist. Bei einer konkreten Ausführungsform hatte die Kamera 3 eine Anzahl von 320 x 256 Pixel, doch wurde diese Zahl in einem anderen Versuch auf 1280 x 1024 erhöht.

Um die Bewegung des Auges 4 sicher erfassen zu können, sind vorzugsweise zwei Lichtquellen 5 vorgesehen, die jedoch bevorzugt Licht in einem nicht sichtbaren Bereich, insbesondere Infrarot, abgeben, um die Sicht des Auges 4 der Testperson nicht zu behindern bzw. zu beeinflussen. Die gezeigte Anordnung der Lichtquellen 5 ist zwar bevorzugt, doch ist die Erfindung nicht darauf beschränkt. Dieses Licht wird dem Auge 4 über einen teildurchlässigen Spiegel 2 zugeführt, wobei dieser Spiegel bevorzugt als dichroïscher Spiegel ausgeführt ist. Somit erfasst die Kamera 3 die Bewegungen des Auges 4 an Hand der Bewegung der Iris (oder eines Teiles derselben), der Pupille, der Sklera etc. Die so gewonnenen Bildsequenzen können dann im Prozessor 6, beispielsweise unter Anwendung einer Autokorrelationsfunktion oder mit einem anderen Bildauswertungs-Algo-rithmus, verarbeitet und die errechnete Auslenkung des Auges in Funktion der Zeit in einem (hier als gesonderte Einheit dargestellten, aber im allgemeinen im Prozessor 6 enthaltenen) Speicher 9 abgespeichert werden. Alternativ erfolgt eine Zwischenspeicherung der Bilder der Kamera 3. In einem weiteren Speicher 8 (alternativ im selben Speicher 9 an dafür reservierten Speicherplätzen) werden die Befehlssignale in Funktion der Zeit abgespeichert, welche der Prozessor 6 (seinerseits gesteuert über den Zufallsgenerator 7) über eine Leitung 10 an den Bildschirm 1 zur Steuerung der Positionen des Stimulus S, S', S" abgibt. Da, wie in jedem Prozessor, ein interner Taktgeber (im einzelnen nicht dargestellt) vorhanden ist, welcher zum Auslesen der Speicher 8, 9 jeweils derselbe ist, können beide Speicher 8, 9 synchron und gleichzeitig ausgelesen und damit die Bewegungen des Stimulus S, S', S" einerseits und des Auges 4 anderseits über die Zeit verglichen werden.

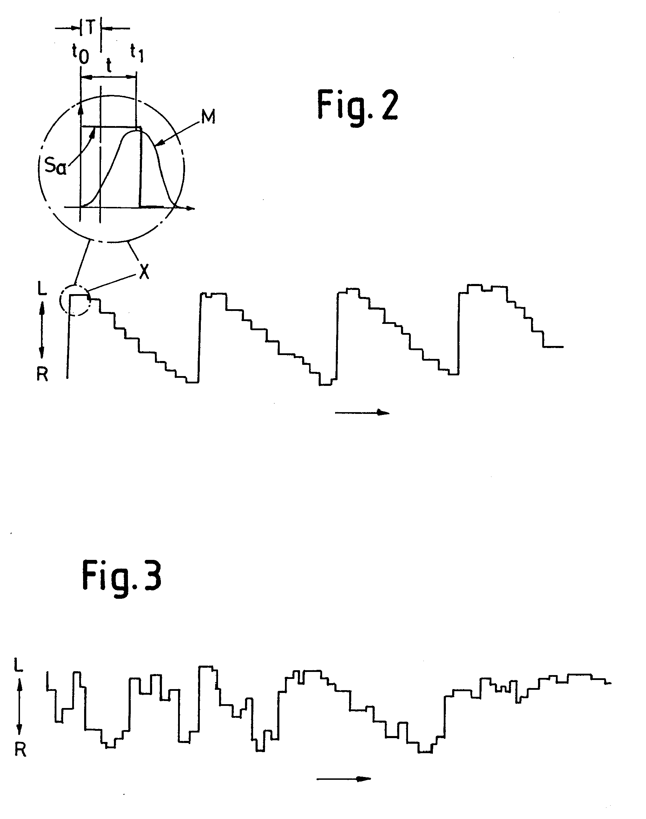

Die Fig. 2 und 3 veranschaulichen an sich Kurven der Bewegung eines Auges, wie sie aus dem Stande der Technik bekannt ist. Dabei wird der Testperson an Stelle eines bewegten Stimulus ein unbewegter in Form eines zu lesenden Textes in mehreren Zeilen gegeben. Das Auge eines unbehinderten Lesers folgt also - entsprechend der Treppenkurve der Fig. 2 - jedem Wort in der Zeile von links nach rechts und kehrt anschliessend in der nächsten Zeile zum Zeilenanfang wieder zurück, so dass dann die nächste Treppenkurve gebildet wird (die horizontale Achse in dieser Darstellung entspricht der Zeitachse).

Bei einem behinderten Leser (Dyslexie) erfolgt jedoch die Augenbewegung nicht so geordnet, sondern etwa in der Art, wie dies die Fig. 3 zeigt. Damit kann man aber erst nach geraumer Weile eine deutliche und signifikante Abweichung der Augenbewegung vom Normalen feststellen, und es bedarf des Lesens mehrerer Zeilen, bevor eine abnormale Abweichung mit Sicherheit diagnostiziert werden kann.

Demgegenüber arbeitet das erfindungsgemässe Verfahren in einem viel kürzeren Zeitbereich, wie sich aus der Vergrösserung des Details X in Fig. 2 ergibt. Dabei entspricht die Treppenkurve Sₐ dem Aufleuchten (Sichtbarkeit) eines Stimulus an einem bestimmten Ort und über eine bestimmte, durch die Breite der Rechteckkurve Sₐ gegebenen Zeit. Sobald der Stimulus in einer vorbestimmten Stelle zum Zeitpunkt t₀ sichtbar wird, folgt ihm das Auge mit einer gewissen Zeitverzögerung. Diese Bewegung des Auges wird nicht ruckartig, sondern etwas verlangsamt erfolgen und ist durch die Kurve M dargestellt. Der Zeitpunkt, in dem das Auge den Stimulus voll erfasst hat, wird etwa der Zeitpunkt t₁ sein. Zwischen t₀ und t₁ liegt der Zeitraum t, der im Normalfall wenige Millisekunden betragen sollte. Bei Personen jedoch, welche durch Einnahme von Alkohol, Medikamenten (wie Analgetika), Drogen oder einfach durch Übermüdung in ihrer Reaktionsfähigkeit eingeschränkt sind, kann der Zeitraum t so lange sein, dass das Auge den Stimulus erst erfasst, wenn dieser bereits wieder seine Position wechselt. Was also hier gemessen wird, ist, verkürzt gesagt, eigentlich die Reaktionszeit (oder die Fähigkeit der Person, auf einen Reiz zu reagieren), und ob diese noch in genügendem Ausmasse vorhanden ist.

Dazu muss natürlich für eine Auswertung der Computer 6 entsprechende Vergleichsdaten gespeichert haben (eigener Speicher oder Verwendung eines Speicherbereiches eines der Speicher 8 oder 9). Ferner ist es klar, dass manche Personen bereits angeboren langsamer sind, weshalb eine gewisse Toleranz einzukalkulieren ist. Der Toleranzbereich T kann statistisch ermittelt werden und wird dann eine fixe Breite innerhalb der vergrösserten Darstellung des Zeitraumes t einnehmen, doch können Umstände gegeben sein, welche es wünschenswert machen, die Toleranzbreite T am Computer (Fig. 1) mittels einer entsprechenden Einrichtung 11 (Tastenfeld, Reglerknopf od.dgl.) einstellbar zu machen. In jedem Falle ist klar, dass bei der an Hand der Fig. 1 gezeigten Organisation der Daten die der Kurve Sₐ entsprechenden Bewegungsdaten des Stimulus S, S', S" dem Speicher 8 entnommen werden können, wogegen die Daten der Bewegung des Auges 4 dem Inhalt des Speichers 9 zu entnehmen sind. Durch Übereinanderlegen dieser Daten im Sinne der vergrösserten Darstellung des Details X der Fig. 2 kann dann die Reak-tionszeit t gewonnen werden.

Am Ende gibt dann der Prozessor 6 ein Testsignal bzw. ein zweckmässig der Reaktionszeit entsprechendes Signal R ab, welches am einfachsten darüber Auskunft gibt, ob die Reaktionszeit sich noch innerhalb des Toleranzbereiches T (Fig. 2) bewegt oder bereits auffällig ist (wonach man die Ursachen gewünschtenfalls in einer nachgängigen Untersuchung eruieren kann). Dieses Testsignal R kann dann auf einem mit dem Prozessor verbundenen Display sichtbar gemacht werden, es kann jedoch gegebenenfalls auch die Auslösung eines akustischen Signales genügen.

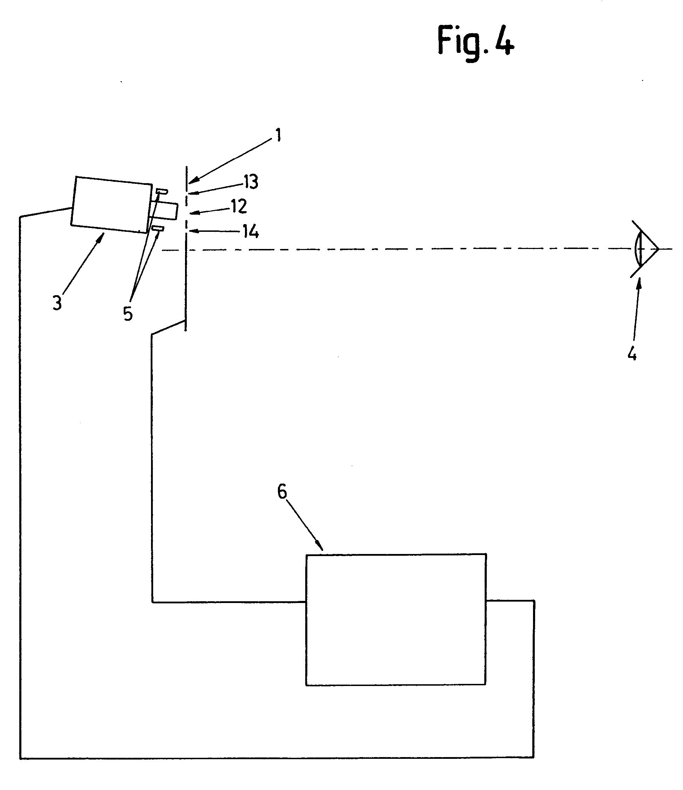

Fig. 4 zeigt eine alternative Ausführungsform der Testanordnung zu jener der Fig. 1. In diesem Falle wird auf den (teuren) dichroïschen Spiegel 2 der Fig. 1 verzichtet. Statt dessen wird das Auge 4 durch ein Loch 12 bzw. einen vom Bildschirm (bzw. den ihn aufbauenden Leuchtdioden) freien Raum von der Kamera 3 beobachtet. Für die Lichtquellen 5 sind entweder entsprechende Löcher 13, 14 vorgesehen, oder sie werden einfach vor dem Bildschirm 1 angeordnet (wobei sie ja im Prinzip von sehr unterschiedlichen Richtungen her das Auge 4 beleuchten können). Die Auswertung über den Prozessor 6 erfolgt in der schon beschriebenen Weise, wobei die einzelnen, mit dem Prozessor verbunden (oder in ihm integrierten) Teile 7-9 und 11 in Fig. 4 nicht dargestellt sind.

Befindet sich also in einem Ausführungsbeispiel des erfindungsgemässen Verfahrens eine zu testende Person vor einem Bildschirm 1 (im weitesten Sinne, wie oben erörtert), dann wird in einem ersten Schritt beispielsweise ein stimulierendes Bild, wie der Punkt S, vor das Auge 4 gebracht.

Gleichzeitig oder erst mit der ersten Bewegung des Stimulus, beispielsweise von S nach S' oder nach S" oder umgekehrt, wird in einem zweiten Schritt die Kamera 3 mit der Augenbeleuchtung 5 in Betrieb genommen (es wurde bereits erwähnt, dass die Augenbeleuchtung 5 zur Vermeidung einer Irritierung oder Beeinflussung des Auges 4 bevorzugt Licht im unsichtbaren Bereich aussendet). Die erste Bewegung des Stimulus erfolgt, wie schon erwähnt, bevorzugt über mindestens etwa 5° (gesehen vom Auge 4 her) und betrug in einem praktischen Beispiel 10° Auslenkung. Ab diesem 2. Schritt läuft die Kamera 3, um eine erste Sequenz von beispielsweise 250 Bilder während 500 ms aufzunehmen. Die Auswahl einer erhöhten Bildaufnahmefrequenz oberhalb von 100 Bildern/sec ist für die Messgenauigkeit sehr wichtig.

Dieser zweite Schritt wird jeweils unter Veränderung der Bewegung des Stimulus vorteilhaft mehrmals wiederholt. Es wurde bereits erwähnt, dass es besonders bevorzugt ist, wenn die Bewegungen des Punktes S, S', S" von einem Auslenkpunkt (bezüglich der Mittellage S oder einer anderen "Null-Position") nicht in einen anderen Auslenkpunkt (S' oder S") übergeht, sondern erst wieder in die "Null-Lage" zurückkehrt. Auf diese Weise erhält man eine Anzahl von Bewegungsstimuli, beispielsweise mindestens 5, vorzugsweise mindestens 10, wobei aber die Anzahl nach oben hin an sich keinen Beschränkungen unterworfen ist, lediglich jenen, die sich aus der Notwendigkeit ergeben, den Test in einer vernünftigen Zeit durchzuführen, was - wie ersichtlich - schon in relativ kurzer Zeit möglich ist. So kann die Anzahl der Bewegungsstimuli auch bis 100 und mehr betragen. All diese Sequenzen der Augenbewegungen werden von der Kamera 3 aufgenommen und in der Auswerteeinrichtung 6 in Vergleich zu den Stimulusbewegungen gesetzt.

Je nach Aufbau der Kamera 3 wird in einem dritten Schritt entweder jede Sequenz sofort auf den Speicher des Computers 6 übertragen, oder die Kamera 3 besitzt selbst einen ausreichend grossen Speicher (es ergeben sich beispielsweise Daten in der Grössenordnung von einigen GB, wie ein praktischer Versuch mit 2,5 GB gezeigt hat), dessen Inhalt erst nach Abschluss an den Computer 6 weitergegeben wird.

Nun erfolgt in einem vierten Schritt die Auswertung, die sich, wie folgt, in mehrere Einzelschritte unterteilt:
1. Zunächst wird, wie bei bekannten Augenbewegungsmessungen auch (beispielsweise zum Fokussieren von Kameras), ein Orientierungsschritt durchgeführt, d.h. der gewonnene Bildinhalt wird analysiert, um gewisse Bildkonturen, -kanten,

-übergänge od.dgl. zu eruieren und zu identifizieren. Obwohl oben bereits gesagt wurde, dass dies etwa an Hand der Iris oder der Sklera erfolgen könnte, ist die Bestimmung der Pupille bevorzugt. Besonders vorteilhaft ist es, wenn der Rand der Pupille mit Hilfe einer kreisförmigen Hough-Transformation bestimmt wird, denn damit erhält man den Kreis der Pupille mit bekanntem Radius und Mittelpunkt.
1. In einem praktischen und vorteilhaften Ausführungsbeispiel legte der Computer eine gedachte horizontale Linie durch den Mittelpunkt dieses Kreises, wobei sich am Umfang je ein Schnittpunkt dieser Linie mit dem Kreis ergab, wobei die so gewonnenen Schnittpunkte ein erstes Paar von zu verfolgenden Punkten bildeten.
2. Man kann die Genauigkeit der Messung gewünschtenfalls in einem Zusatzschritt noch etwas erhöhen, indem man sich mit diesen zwei Punkten nicht zufrieden gibt, sondern die horizontale Linie weiter verfolgt, um die Grenze zwischen Iris und Sclera (Limbus) zu suchen und damit zwei weitere Punkte zu gewinnen. Algorithmen zur Ermittlung dieser Übergänge bzw. Kanten eines Bildes sind an sich in grosser Zahl bekannt. Bevorzugt (und am genauesten) ist ein auf Differentiation beruhender Kanten-Detektionsalgorithmus. So erhält man dann - wenigstens in der der "Null-Lage" (siehe S in Fig. 1) entsprechenden Stellung des Auges - insgesamt vier Punkte (links und rechts am Pupillenrand sowie links und rechts am Limbus).
3. Die Bildauswertung nach den obigen Einzelschritten b) bzw. c) kann an Hand mehrerer Bildsequenzen durchgeführt werden, wenn eine einzige Sequenz unbestimmt sein sollte, doch wird es im allgemeinen so sein, dass man an Hand der ersten Sequenz all diese Punkte bestimmen kann. Daher kann in jedem folgenden Bild die Position der zwei bzw. vier Punkte - jeweils unter Anwendung des Kanten-Detektionsalgorithmus - der Bewegung des Auges nachgeführt. So erhält man für jede Sequenz vier Koordinaten-Reihen, welche die Position in der Horizontalen (es wurde bereits erwähnt, dass die Stimulusbewegung in der Horizontalen bevorzugt ist) der jeweils zu verfolgenden Punkte in Funktion der Zeit beschreiben (vgl. Fig. 2).
4. Da die Abbildungsgeometrie bekannt ist, können die im Einzelschritt d) gewonnenen Koordinaten in Winkel umgerechnet werden. Der dafür benötigte, a priori unbekannte Augendurchmesser (d.h. nicht der Durchmesser der Pupille selbst, der ja bereits im Einzelschritt a) ermittelt wurde) kann fürs erste geschätzt werden. Gewünschtenfalls kann dann diese Schätzung mit an sich bekannten Methoden anschliessend noch genauer gemacht werden.
5. So erhält man die Werte für die Augenbewegung in Funktion der Zeit, beispielsweise in Form von Kurven. Diese Werte bzw. Kurven werden dann in einem weiteren Einzelschritt so ausgewertet, dass (vgl. Fig. 2) die Latenz-Zeit T (d.i. die Zeit zwischen dem Beginn der Bewegung des Stimulus t₀ und dem Beginn der Auslenkung des Auges Sₐ) und allenfalls die Drehgeschwindigkeit des Auges ermittelt wird. Gewünschtenfalls lassen sich auch höhere Ableitungen der Augenbewegung zur Auswertung heranziehen, doch wird im allgemeinen die Zeit T ausreichen.
6. Nun kann die Zeit T (oder auch weitere Bewegungsdaten des Auges) mit einem Toleranzbereich verglichen werden. In jedem Falle erfolgt nach dem Einzelschritt f) eine Speicherung und/oder eine entsprechende Anzeige. Dies kann in Form eines Ausdruckes auf Papier oder einer Darstellung auf einem Display erfolgen. Vorteilhaft ist die Angabe eines "Scores", d.h. einer Unterteilung des möglichen Variationsbereiches in Zeitverschiebungen, die in einem Bereich von "sehr gut", "gut" etc. liegen, oder man gibt einen zahlenmässigen "Score" an, um jedenfalls so auch ungeschultes Personal auf einfache Weise zu informieren, wie die Fahrtüchtigkeit bzw. die Reaktionsfähigkeit der getesteten Person liegt. Ist diese Fahrtüchtigkeit bzw. Reaktionsfähigkeit ungenügend, dann wird es angezeigt sein, dies durch weitere, länger dauernde Tests auch zu belegen. Zum Vergleich sei bemerkt, dass der erfindungsgemässe Test im allgemeinen kaum länger als fünf Minuten dauern wird und doch bereits eine aussagekräftige Beurteilung über den Fahrtüchtigkeitszustand bzw. das Reaktionsvermögen einer getesteten Person zulässt.

Im Rahmen der Erfindung sind zahlreiche Varianten denkbar. Beispielsweise ist zwar die Messung der Reaktionszeit t die einfachste und für den Zweck signifikanteste, doch ist das erfindungsgemässe Verfahren auch beispielsweise für hirngeschädigte Patienten oder in anderen neurologisch zu untersuchenden Fällen anwendbar, wobei dann es unter Umständen darauf ankommt, welchen 2-dimensionalen Verlauf die Augenbewegung (dazu ist ein zweidimensionaler "Bildschirm" vorteilhaft) nimmt, d.h. ob sie direkt auf den die Position wechselnden Stimulus blickt oder dies über Umwege tut. In diesem Falle kann der 2-dimensionale Bewegungsverlauf des Stimulus, demjenigen des Auges gegenübergestellt und aus den Abweichungen ein entsprechendes Testsignal gewonnen werden. Statt der Verwendung einer Kamera 3 mag es für einfachere Anordnungen auch ausreichend sein, mehrere lichtempfindliche Elemente (Photodioden, Phototransistoren), z.B. in einem Array, zu verwenden. Auch wurde eingangs bereits darauf hingewiesen, dass sich der Test für alle Personen eignet, die einer starken Beanspruchung, wie etwa beim Hochleistungssport, bei Erbringung einer Leistung unter extremen Umständen, wie Besteigung von Bergen extremer Höhe etc. unterworfen sind. Hat man eine NORMAL-Reaktionszeit definiert oder bei einer bestimmten Person gemessen, dann kann eine Überreaktion, wie sie etwa in Doping-Fällen vorkommen kann, mit Hilfe des erfindungsgemässen Verfahrens ebenfalls bestimmt werden. Hier wird also der Ausdruck "neurologische Beeinträchtigung" in seinem allgemeinsten Sinne gebraucht.

### Unerkannter Text

## Patentansprüche

1. Verfahren zum Bestimmen neurologischer Beeinträchtigungen, bei der eine Augenbewegung einer zu testenden Person mittels eines optischen Stimulus (S, S', S") stimuliert wird, die Augenbewegung ermittelt und in ein entsprechendes elektronisches Signal umgesetzt wird und aus der Augenbewegung nach einem Vergleich ein Testsignal (R) gewonnen wird, **dadurch gekennzeichnet, dass** ein in seiner Position veränderlicher Stimulus (S, S', S") verwendet wird, dem das Auge (4) folgen soll, und dass das Testsignal (R) aus dem Vergleich der Bewegung des Stimulus (Sₐ) mit der Bewegung (M) des Auges (4) gewonnen wird.

2. Verfahren nach Anspruch 1, **dadurch gekennzeichnet, dass** bei dem Vergleich der Zeitabstand (t) zwischen den beiden Bewegungen (Sₐ, M) ermittelt und für das Testsignal herangezogen wird.

3. Verfahren nach Anspruch 1 oder 2, **dadurch gekennzeichnet, dass** das stimulierende Bild (S, S', S") wenigstens einer der folgenden Anforderungen genügt: a) dass es über die Zeit mindestens drei verschiedene Positionen einnimmt, vorzugsweise über eine zweidimensionale Darstellungsfläche, b) dass die Positionen des stimulierenden Bildes jeweils wenigstens annähernd in einer Horizontalen liegen, c) dass das stimulierende Bild (S, S', S") relativ kleinflächig, insbesondere im wesentlichen punktförmig ist, d) die Abstände der Positionen des Stimulus (S, S', S") einer Sehwinkelveränderung des Auges (4) von wenigstens 3°, insbesondere von wenigstens 5°, z.B. 10°, entsprechen, e) der Stimulus (S, S', S") ist auf einem sich verändernden, beispielsweise bewegten, Hintergrundmuster dargestellt, f) der Kontrast des Stimulus (S, S', S") zu seinem Hintergrund, insbesondere zu einem Hintergrundmuster, ist in verschiedenen Positionen unterschiedlich, beispielsweise durch Helligkeitsveränderung des Stimulus (S, S', S").

4. Verfahren nach einem der vorhergehenden Ansprüche, **dadurch gekennzeichnet, dass** die Position und/oder die Darstellungsdauer des stimulierenden Bildes (S, S', S") einer Zufallsverteilung unterworfen wird.

5. Verfahren nach einem der vorhergehenden Ansprüche, **dadurch gekennzeichnet, dass** eine Anzahl von Sequenzen von Stimulusbewegungen durchgeführt wird und jede Sequenz gespeichert wird.

6. Vorrichtung zur Durchführung des Verfahrens nach einem der vorhergehenden Ansprüche, mit einer ein stimulierendes Signal (S, S', S") abgebenden Einrichtung (1, 6, 7), einer die Augenbewegung beobachtenden, ein entsprechendes Ausgangssignal liefernden Einrichtung (3), einer Vergleichs- und Auswerteeinrichtung (6), **dadurch gekennzeichnet, dass** der ein stimulierendes Signal abgebenden Einrichtung (1, 6, 7) ein Bewegungs-und/oder Kontrastsignal zuführbar ist, dass der Vergleichs- und Auswerteeinrichtung (6) sowohl das genannte Ausgangssignal als auch das Bewegungs- bzw. Kontrastsignal zuführbar ist, wobei die daraus gewonnenen Unterschiede das Testsignal (R) bilden.

7. Vorrichtung nach Anspruch 6, **dadurch gekennzeichnet, dass** die die Augenbewegung beobachtende Einrichtung eine Video-Aufzeichnungseinrichtung, insbesondere eine Kamera (3) mit einer grösseren Bildfrequenz als 100 Bildern/s, vorzugsweise mindestens 250 Bildern/s und insbesondere in einem Bereich von 400 bis 600 Bildern/s, z.B. 500 Bildern/s, umfasst, der vorzugsweise eine Bildauswerte-Software zugeordnet ist.

8. Vorrichtung nach Anspruch 6 oder 7, **dadurch gekennzeichnet, dass** die das stimulierende Signal abgebende Einrichtung (1, 6, 7) wenigstens eines der folgenden Merkmale aufweist: a) einen Bildschirm (1) zur Darstellung eines bewegten Bildes und/oder eines, beispielsweise bewegten, Hintergrundmusters; b) mehrere Lichtquellen, insbesondere Leuchtdioden.

9. Vorrichtung nach einem der Ansprüche 6 bis 8, **dadurch gekennzeichnet, dass** ein Zufallsgenerator (7) zum Steuern des Bewegungssignals vorgesehen und mit der Vergleichs- und Auswerteeinrichtung (6) verbunden ist.

10. Vorrichtung nach einem der Ansprüche 6 bis 9, **dadurch gekennzeichnet, dass** eine Einstelleinrichtung (11) für die Toleranzbreite (T) vorgesehen und mit der Vergleichs- und Auswerteeinrichtung (6) verbunden ist.
